# FASCICULE DE BREVET EUROPEEN

(11) **EP 1 988 947 B1**
(45) Date de publication et mention de la délivrance du brevet: **18.11.2009**
(21) Numéro de dépôt: 07701894.3
(22) Date de dépôt: 26.02.2007
(51) Int. Cl.: A61M 1/36, B04B 5/04

(54) **Procédé et chambre de centrifugation pour le lavage et la séparation en continu de constituants sanguins**
Zentrifugationsverfahren und Kammer zum Waschen und kontinuierlichen Trennen von Blutbestandteilen
Centrifugation method and chamber for wasching and continuous separation of blood constituents

(30) Priorité: 28.02.2006 EP 06405087
(43) Date de publication de la demande: 12.11.2008
(73) Titulaire: Rochat, Jean-Denis, 1272 Genolier (CH)
(72) Inventeur: MOSIMANN, Laurent, CH-1291 Commugny (CH)
(74) Mandataire: Savoye, Jean-Paul
(86) Numéro de dépôt international: PCT/CH2007/000094
(87) Numéro de publication internationale: WO 2007/098623

(56) Documents cités:
- US-A- 5 445 593
- US-A- 5 971 948

## Description

La présente invention a pour objet un procédé et une chambre de centrifugation pour le lavage et la séparation en continu de constituants sanguins, en particulier de globules rouges à partir de sang lavé, destinée à l'autotransfusion lors d'opérations ou de saignements post-opératoires notamment, mais pouvant également être utilisée pour des opérations de déglycérolisation par exemple.

Le principe le plus communément utilisé pour laver et séparer les constituants sanguins est la centrifugation. Sous l'effet de la force centrifuge, les différents constituants sanguins se séparent en raison de leurs densités inégales. Cette séparation s'effectue naturellement dans un ordre prédéfini de sorte que le constituant sanguin de plus grande densité, à savoir les globules rouges, se positionnent toujours à la plus grande distance possible de l'axe de révolution de la chambre, alors que les autres constituants naturels ou ajoutés de plus faible densité, respectivement comme le plasma ou une solution stérile de lavage, se situeront toujours plus proche de cet axe que tous les autres.

L'utilisation d'automates d'autotransfusion permet de récupérer le sang saigné pendant ou après une opération par aspiration au moyen d'une canule. Le sang aspiré est ensuite mélangé à une solution anti-coagulante puis est stocké provisoirement dans un réservoir filtrant les particules indésirables les plus grossières telles que caillots sanguins, débris osseux ou tissus organiques. Il est ensuite pompé dans une chambre de centrifugation. Les globules rouges sont dans un premier temps concentrés par centrifugation puis lavés grâce à une injection d'une solution stérile telle qu'une solution saline par exemple. Les composants indésirables comme le plasma, les déchets, les stromas et la solution stérile sont ensuite extraits de la chambre de centrifugation après sédimentation et collectés dans une poche destinée à être jetée, alors que le concentré lavé de globules rouges est transféré dans une poche de re-injection faisant office de réservoir tampon avant de retourner au patient.

Le document US 5'445'593 décrit un dispositif de lavage et de séparation permettant de réaliser un lavage en continu. Suivant ce procédé, le pompage du sang dans la chambre du séparateur centrifuge, l'injection de la solution stérile dans le sang pompé, l'extraction des déchets de lavage issus de la centrifugation ainsi que l'extraction du concentré globulaire sont autant d'opérations qui sont réalisées simultanément. La chambre de ce dispositif est constituée d'un canal annulaire en spirale de section rectangulaire dont les deux sections d'extrémités sont fermées. Ce canal est subdivisé dans sa longueur en trois zones respectivement destinées à une première séparation des constituants sanguins, à mélanger ces constituants avec une solution de lavage injectée dans la seconde zone-, et à une seconde séparation durant laquelle le concentré globulaire sera extrait de la chambre. Les entrées et sorties utiles pour l'injection et l'extraction du sang, de ses constituants ainsi que de la solution de lavage sont situées sur les parois extérieures de cette chambre ce qui nécessite l'agencement de tuyaux tournant à la périphérie de cette chambre à la manière d'un lasso. Cette-manière de faire rend la réalisation de chambre complexe, accroît son encombrement et ne facilite pas la préparation et l'utilisation de ce dispositif pour le personnel médical.

De plus, l'injection de la solution de lavage telle que proposée dans ce dispositif de l'art antérieur crée inévitablement des turbulences qui d'une part favorisent le re-mélange des constituants sanguins déjà partiellement séparés, et d'autre part ne permet pas d'utiliser pleinement la capacité nettoyante de la solution de lavage. En effet, en raison de son injection radiale, de sa faible section d'injection et de sa faible densité, la solution de lavage aura tendance à traverser rapidement la couche de sang sous l'effet de la force centrifuge, jusqu'à venir surnager à la surface de l'écoulement sanguin en se positionnant plus près de l'axe de rotation que n'est le sang. Pour ces mêmes raisons, le déplacement radial de cette solution de lavage ne s'effectue que localement dans une zone peu étendue.

Le but de la présente invention vise à remédier au moins .en partie aux inconvénients précités en suggérant un procédé et une chambre de centrifugation pour le lavage et la séparation en continu de constituants sanguins suivant un ratio Volume de solution de lavage/Volume de globules rouges traités qui soit très faible, notamment grâce à une bonne répartition du flux de la solution de lavage. Un autre but visé consiste à obtenir une chambre de centrifugation qui soit peu encombrante et de mise en oeuvre simple.

A cet effet, la présente invention a pour objet un procédé ainsi qu'une chambre de centrifugation conforme à ce qu'énoncent les revendications 1 et 2 respectivement.

Avantageusement, ce procédé et cette chambre de centrifugation permettent une économie substantielle de solution stérile de lavage contribuant ainsi à réduire les coûts des traitements médicaux pour lesquels cette chambre est destinée, ainsi qu'à simplifier les manipulations effectuées par le personnel médical.

D'autres avantages apparaîtront à la lumière de la description qui va suivre et qui se réfère à un mode de réalisation préféré de l'objet de la présente invention, pris à titre nullement limitatif et illustré par les figures annexées dans lesquelles:
La figure 1 est une vue schématique en demi-coupe verticale de la chambre de la présente invention représentée sans aucun flux sanguin ni solution de lavage.
La figure 2 est une vue identique à celle de la figure 1 mais dans laquelle les flux de sang et de solution de lavage sont représentés suivant des conditions normales d'utilisation.
Les figures 3 et 4 sont des vues de détail de la figure 2, respectivement de sa partie supérieure et de sa partie inférieure.

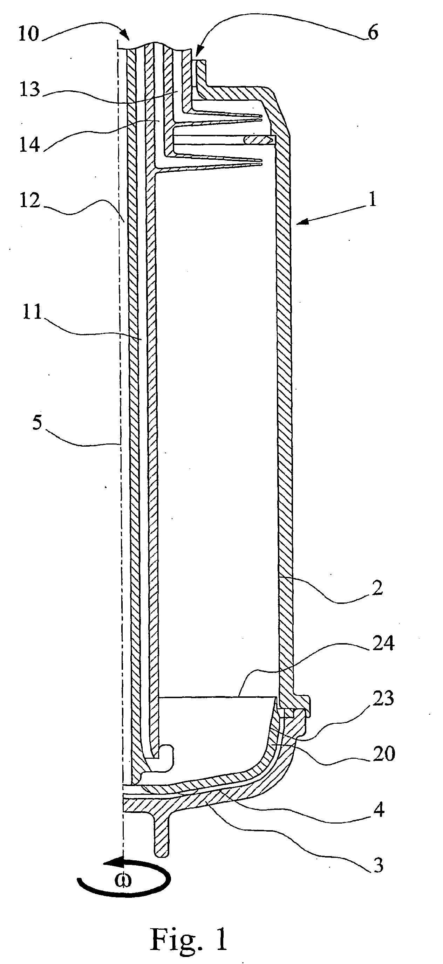

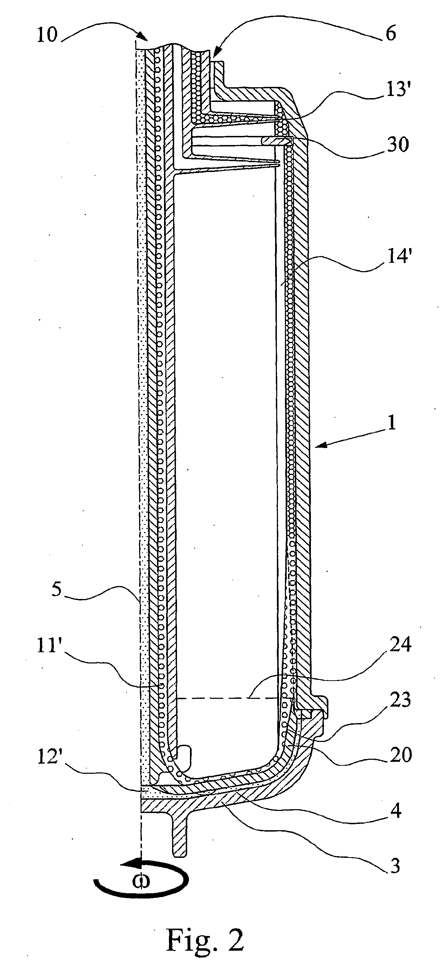

La chambre 1 de centrifugation, telle qu'illustrée aux figures 1 et 2, comprend une paroi latérale cylindrique intérieure 2, un fond 3 présentant une paroi intérieure 4, et adopte de préférence une forme cylindrique allongée autour de son axe de révolution 5. Son extrémité supérieure présente une ouverture circulaire 6 pourvue d'un moyen d'étanchéité non représenté, pouvant se présenter sous la forme d'un joint rotatif. Ce moyen d'étanchéité vise à garantir la stérilité à l'intérieur de la chambre en entourant un organe statique 10 d'admission/évacuation s'étendant dans la chambre, au moins partiellement le long de son axe de révolution 5.

La partie fixe que constitue cet organe statique 10 comprend une pluralité de conduits permettant d'effectuer aisément des échanges de fluides entre l'intérieur et l'extérieur de la chambre de centrifugation. Parmi les conduits visant à apporter des fluides dans la chambre, il est prévu que l'organe statique 10 comprenne au moins un premier canal d'amenée 11 pour l'entrée, du sang 11' dans la chambre et au moins un second canal d'amenée 12 pour l'adduction simultanée d'une solution stérile de lavage 12' telle qu'une solution saline par exemple. Afin de pouvoir extraire ces fluides de la chambre une fois traités et autoriser une circulation en continu du flux sanguin, il est prévu que l'organe statique 10 soit également pourvu d'au moins un premier canal de sortie 13 pour au moins un constituant 13' de ce sang, tel qu'un concentré de globules rouges lavés, et au moins un second canal de sortie 14 pour extraire de la chambre un fluide 14' formé d'une part des autres constituants sanguins, comme le plasma par exemple, et d'autre part de la solution de lavage chargée de déchets issus du sang traité.

Selon le mode de réalisation préféré de l'invention, le premier canal d'amenée 11, destiné à l'introduction du sang 11' à traiter, possède une forme annulaire qui entoure le second canal d'amenée 12. De préférence, les canaux d'amenée 11 et 12 sont concentriques et le second canal d'amenée est situé sur l'axe de rotation 5 de la chambre. Les extrémités aval de ces deux canaux se situent à proximité du fond 3 de cette chambre, plus précisément à proximité de la paroi interne 4 de ce fond.

L'extrémité aval du second canal d'amenée 12 est prolongée à sa périphérie par un déflecteur circulaire 20 qui s'étend, à distance de la paroi intérieure 4, dans le fond 3 de la chambre. Ce déflecteur circulaire 20 constitue pour la chambre de centrifugation une sorte de double fond s'étendant de manière sensiblement parallèle à la paroi intérieure 4 avant de remonter, sur son bord extérieur partiellement le long de la paroi latérale cylindrique intérieure 2.

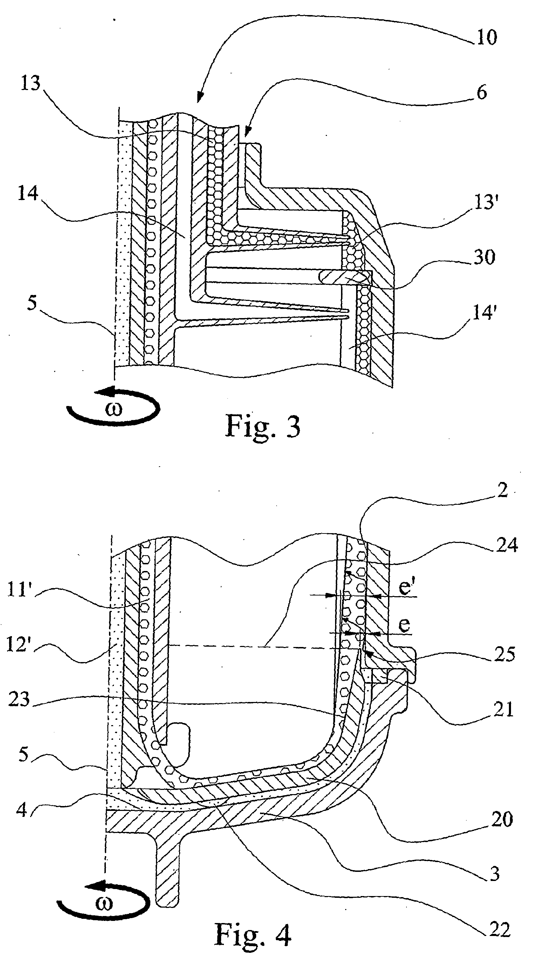

Selon le mode de réalisation préféré, et comme mieux visible sur la figure 4 illustrant en détail la partie inférieure de la chambre représentée à la figure 2, on remarque que le fond 3 de cette chambre constitue la partie inférieure de celle-ci et qu'elle est rendue solidaire du bas de la paroi latérale 2 par un système d'assemblage conventionnel tel qu'un emboîtement, un clipsage ou une soudure par exemple. Ce système d'assemblage a pour bénéfice de pouvoir également être utilisé pour y insérer des ailettes de fixation 21 pour le maintient du déflecteur cylindrique 20 dans sa position telle que décrite ci-avant. Aux abords de son extrémité aval ce déflecteur repose sur une pluralité de bossages 22 répartis de façon égale sur sa circonférence. Ces bossages font office de points d'appui et sont de préférence aussi fins que possible pour ne pas perturber l'écoulement du liquide stérile de lavage 12'. Le déflecteur circulaire 20 vise à déterminer, avec la paroi intérieure 4 du fond 3 de la chambre, un passage pour l'écoulement de la solution stérile de lavage 12' qui soit séparé de celui du sang 11' . L'écoulement de ce dernier se faisant sur la face supérieure 23 du déflecteur 20 et s'y trouve instantanément plaqué en raison de l'effet que porte la force centrifuge sur ce fluide.

A son extrémité aval, le-déflecteur circulaire 20 se termine le long de la paroi latérale cylindrique intérieure 2 par une arête 24 qui définit, avec cette paroi, une section annulaire de passage 25 pour l'écoulement de ladite solution stérile de lavage. Cette arête constitue l'extrémité d'un déversoir du déflecteur circulaire 20. Elle est de préférence située à un niveau compris dans le premier quart inférieur de la hauteur de la chambre de centrifugation. Comme mieux visible sur la figure 4, cette section annulaire de passage 25 possède une épaisseur e sensiblement inférieure à l'épaisseur locale e' de la couche de sang 11' en rotation dans la chambre 1 au niveau de ladite arête 24. De préférence, cette section annulaire de passage possède une épaisseur comprise entre 0.02 mm et 2 mm et le niveau de l'arête 24 est distant de 5 à 50 mm du niveau de l'extrémité aval du second canal d'amenée 12.

Grâce à ce mode de réalisation, l'injection du flux de la solution stérile de lavage 12' s'effectue avantageusement dans le même sens que le courant ascendant du sang 11'. De plus, cette injection s'effectue à la périphérie du lit sanguin, à savoir le long de la paroi latérale cylindrique intérieur 2 de la chambre. De ce fait, les remous et remélanges que pourrait engendrer une telle injection au sein de la couche de sang déjà partiellement sédimentée sont réduits autant que possible. Ceci permet d'éviter toute formation de by-passes dans la couche de sang, à savoir toute formation de ponts de solution stérile qui traverseraient l'épaisseur de cette couche et qui court-circuiteraient l'effet de lavage que l'on souhaite optimiser lors de l'injection de cette solution.

La formation de by-passes est également liée à la vitesse de pénétration du flux de la solution de lavage à la périphérie de la couche de sang au sein de la chambre de centrifugation. Si cette vitesse est faible alors l'épaisseur de la couche de solution de lavage, correspondant à la épaisseur e de la section annulaire de passage 25, sera nécessairement élevée et les risques de by-passes accrus. En revanche, si la vitesse de pénétration du flux de la solution de lavage est élevée, et que par conséquent la épaisseur e est faible, alors le même volume de solution de lavage se répartira sur une plus grande surface de sang à laver, ce qui réduira le risque de formation de by-passes.

Outre l'absence de formations de by-passes pour les raisons évoquées ci-dessus, le ratio volume solution de lavage/Volume de globules rouges traités est encore amélioré par le fait que la épaisseur e de la section annulaire de passage 25 est très fine et que cette section de passage permet de créer une sorte de filet ou de film annulaire de solution stérile de lavage qui se trouve initialement plaqué contre la paroi latérale cylindrique intérieur de la chambre au niveau de l'arête 24 du déflecteur. De préférence, le rapport Surface/Epaisseur de la solution stérile de lavage sur la paroi latérale cylindrique intérieur 2 est très élevé.

Durant la centrifugation, trois effets simultanés vont se produire. D'une part, la solution stérile de lavage injectée sous forme d'un film annulaire uniforme de faible épaisseur e va traverser, sous l'effet de la force centrifuge, l'épaisseur e' du flux sanguin 11' sur toute sa longueur et emporter avec elle les impuretés jusqu'à ce que cette solution se retrouve à la surface du sang ainsi lavé. D'autre part, le plasma va être repoussé en direction du centre de la chambre de centrifugation, emmené par le déplacement concentrique de cette solution de lavage. Enfin, la sédimentation simultanée des globules rouges va accélérer l'effet de flux croisés obtenu entre la solution de lavage et les globules rouges.

Cette migration de la solution stérile de lavage va s'effectuer progressivement en débutant à la sortie aval du déflecteur 3 et en se terminant plus haut, aux abords de la partie médiane ou supérieure de la hauteur de la chambre, mais dans tous les cas avant d'atteindre le niveau du plus bas des collecteurs reliés aux canaux de sortie 13, 14. Ainsi, la solution de lavage va en quelque sorte pouvoir filtrer le flux sanguin plaqué contre la paroi latérale de la chambre en le traversant régulièrement dans toute son épaisseur. De cette manière, on évitera toute formation de by-passes et on optimisera l'effet de lavage de la solution stérile. Le ratio susmentionné de cette solution s'en trouvera alors optimisé.

En référence à la figure 3, celle-ci montre en détail la partie supérieure de la chambre 1 telle qu'illustrée à la figure 2. A ce niveau, la sédimentation par effet centrifuge du sang 11' est terminée tout comme son lavage au moyen de la solution stérile 12'. Ce sang 11' initialement chargé d'impuretés se sera séparé en au moins deux couches distinctes de fluides 13' et 14' respectivement situées contre la paroi latérale cylindrique intérieure 2 de la chambre et à une distance plus proche de l'axe de révolution de cette chambre. Le premier fluide 13' de plus grande densité correspond au concentré de globules rouges qui, par exemple, sera ré-injecté au patient selon le principe d'une auto-transfusion, alors que le second fluide 14' de moindre densité comprend les autres constituants sanguins, dont au moins le plasma, ainsi que tous les déchets qui on été retirés du sang 11' par la solution stérile de lavage.

Le concentré globulaire 13' est extrait de la chambre, dans sa partie la plus haute, au moyen d'un collecteur constituant la partie terminale de l'extrémité amont du premier canal de sortie 13. Il est ensuite collecté dans une poche intermédiaire de stockage, non représentée, faisant office de volume tampon avant d'être ré-injecté au patient. D'une manière similaire, le fluide 14' est extrait de la chambre au moyen d'un autre collecteur situé à l'extrémité amont du second canal de sortie 14 pour être finalement collecté dans une poche, non représentée, qui sera jetée. Les extrémités amont de ces collecteurs se trouvant plongées dans l'épaisseur de chaque couche de fluide pour lesquels ces collecteurs sont destinés.

Suivant une forme d'exécution préférée, un barrage circulaire 30, de préférence en forme d'anneau plat concentrique à l'axe de révolution de la chambre de centrifugation, est disposé entre les collecteurs des canaux de sortie 13, 14 pour empêcher le re-mélange des deux fluides 13' 14' lors de leur extraction. A cet effet, le diamètre externe de ce barrage circulaire 30 est supérieur au diamètre de l'interface entre la couche de globules rouges 13', qui est la couche la plus éloignée de l'axe de révolution de la chambre, et la couche du fluide 14' constituées par les autres constituants sanguins et par la solution de lavage 12' chargée de déchets. Le diamètre interne d'un tel barrage annulaire est inférieur au diamètre interne de la couche de fluide 14' la plus proche de l'axe de révolution de la chambre. Grâce à la séparation que constitue ce barrage, les collecteurs des canaux de sortie 13 et 14 peuvent fonctionner de manière indépendante, les éventuelles perturbations de flux créées par l'un n'affectant pas le second.

De préférence, le débit d'entrée du sang 11' dans la chambre de centrifugation 1 est régulé en fonction de son taux d'hématocrite de manière à ce que le flux entrant de globules rouges soit constant. Cette régulation, non représentée mais pouvant être réalisée par un moyen, de pompage ou par une valve de régulation, est pilotée par une mesure du taux d'hématocrite du sang 11' en amont de la chambre de centrifugation.

De préférence encore, le flux de la solution stérile de lavage 12' est continu et proportionnel au flux interstitiel de sortie des globules rouges 13' lavés et re-concentrés. Typiquement, le débit du flux de la solution de lavage peut se situer dans un rapport compris entre 1 à 10 fois celui du flux de globules rouges traités.

Suivant un autre mode de réalisation possible, l'arête 24 du déflecteur circulaire 20 est située à un niveau compris dans le tiers ou la moitié inférieure de la hauteur de la chambre de centrifugation. De cette manière la solution stérile de lavage 12' sera injectée dans une couche de sang 11' d'épaisseur locale e' plus faible mais qui avantageusement sera à un stade de sédimentation plus avancé. De ce fait cette couche comprendra à ce niveau une plus forte concentration de globules rouges, ce qui nécessitera une quantité moindre de solution de lavage. A ce propos, il convient toutefois de noter que l'injection de la solution stérile de lavage sous la forme d'un film annulaire requière une certaine fluidité de la masse de globules rouges pour éviter la dislocation prématurée de ce film et générer ainsi la formation de by-passes. Ainsi, le niveau auquel doit se trouver l'arête 24 pour pouvoir optimiser le rendement de la solution stérile de lavage, résultera notamment d'un compromis entre la viscosité de la masse globulaire partiellement sédimentée du sang 11' et l'épaisseur du film de la solution de lavage au droit de cette arête. Cette épaisseur correspondant à l'épaisseur e de la section annulaire de passage 25.

Suivant une autre forme d'exécution non illustrée, la section annulaire de passage 25 pourrait être constituée d'une bague, couronne ou anneau en matière poreuse, dont l'ouverture des pores serait de préférence trop faible pour laisser passer les globules rouges mais suffisamment grande pour autoriser le passage de la solution stérile de lavage. L'agencement d'une bague en matière poreuse à l'endroit correspondant à la section annulaire de passage 25 permettrait d'allonger la surface de mise en contact entre la solution stérile de lavage et le sang ou le concentré de globules rouges.

Les dimensions de la chambre de centrifugation sont de l'ordre de 50 à 200 mm de hauteur pour un diamètre compris entre 15 et 150 mm environ.

Du principe de l'effet de flux croisés, obtenu entre la solution de lavage et les globules rouges, découle le procédé de la présente invention. Ce dernier est destiné au lavage et à la séparation en continu de constituants sanguins à partir de sang 11' et d'une solution stérile de lavage 12'. De densité inférieure à celle du sang 11', cette solution est, avec le sang 11', introduite dans la chambre 1 de centrifugation. La paroi latérale cylindrique intérieure 2 de cette chambre 1 se trouvant en rotation autour de l'axe de révolution 5.

Selon l'invention, on forme un écoulement tubulaire axial de sang 11' et d'épaisseur locale e' contre la paroi latérale cylindrique intérieure 2. On forme également, entre cette paroi latérale cylindrique intérieure 2 et l'écoulement tubulaire axial de sang 11', un film de solution stérile de lavage 12' d'épaisseur e sensiblement inférieure à l'épaisseur locale e'. Ce film s'écoule continuellement dans la même direction axiale que l'écoulement tubulaire de sang 11' de façon à ce que, sous l'effet de la force centrifuge, il traverse progressivement l'épaisseur e' de sang 11' en direction de l'axe de révolution 5 de la chambre 1. Ainsi, le déplacement radial de ce film s'effectue depuis son contact avec la paroi latérale cylindrique intérieure 2 jusqu'à la surface de l'écoulement tubulaire de sang 11' la plus proche de l'axe de révolution 5. De préférence, le sens de l'écoulement tubulaire axial de sang 11' est ascendant et le film de solution stérile de lavage est alors obtenu en injectant cette dernière dans ce sens ascendant, à la surface de la paroi latérale cylindrique intérieure 2.

## Revendications

1. Procédé pour le lavage et la séparation en continu de constituants sanguins à partir de sang (11') et d'une solution stérile de lavage (12'), de densité inférieure à celle du sang (11'), introduits dans une chambre (1) de centrifugation pourvues d'une paroi latérale cylindrique intérieure (2) en rotation -autour d'un axe de révolution (5), **caractérisé en ce qu'**on forme un écoulement tubulaire axial de sang (11') et d'épaisseur locale (e') contre la paroi latérale cylindrique intérieure (2), et on forme entre cette dernière et ledit écoulement tubulaire axial de sang (11') un film de solution stérile de lavage (12') et d'épaisseur (e) sensiblement inférieure à l'épaisseur locale (e'), s'écoulant dans la même direction axiale que l'écoulement tubulaire de sang (11') de façon à ce que, sous l'effet de la force centrifuge, ce film traverse progressivement l'épaisseur (e') de sang (11') en direction de l'axe de révolution (5) de la chambre (1).

2. Chambre (1) de centrifugation pour le lavage et la séparation en continu de constituants sanguins, comprenant une paroi latérale cylindrique intérieure (2), un fond (3) présentant une paroi intérieure (4), un axe de révolution (5) ainsi qu'une ouverture circulaire (6) pourvue d'un moyen d'étanchéité disposé autour d'un organe statique (10) d'admission/évacuation s'étendant dans ladite chambre (1) au moins partiellement le long de son axe de révolution (5) et comprenant au moins un premier canal d'amenée (11) pour le sang (11'), au moins un second canal d'amenée (12) pour apporter simultanément une solution stérile de lavage (12'), au moins un premier canal de sortie (13) d'au moins un constituant (13') de ce sang et au moins un second canal de sortie (14) pour un fluide (14') formé de ladite solution de lavage chargée de déchets et des autres constituants sanguins, **caractérisée en ce que** le second canal d'amenée (12) a son extrémité aval prolongée à sa périphérie par un déflecteur circulaire (20) qui s'étend dans le fond (3) de la chambre (1) à distance de la paroi intérieure (4) et se termine le long de la paroi latérale cylindrique intérieure (2) par une arête (24) qui définit, avec cette paroi, une section annulaire de passage (25) pour l'écoulement de ladite solution stérile de lavage (12').

3. Chambre (1) de centrifugation selon la revendication 2, **caractérisée en ce que** le premier canal d'amenée (11) est annulaire et entoure le second canal d'amenée (12).

4. Chambre (1) de centrifugation selon la revendication 3, **caractérisée en ce que** les premier et second canaux d'amenée (11, 12) sont concentriques.

5. Chambre (1) de centrifugation selon la revendication 2, **caractérisée en ce que** la section annulaire de passage (25) possède une épaisseur (e) sensiblement inférieure à l'épaisseur locale (e') d'une couche de sang (11') en rotation dans la chambre (1) au niveau de ladite arête (24).

6. Chambre (1) de centrifugation selon la revendication 5, **caractérisée en ce que** la section annulaire de passage (25) possède une épaisseur (e) comprise entre 0.02 mm et 2mm.

7. Chambre (1) de centrifugation selon la revendication 5, **caractérisée en ce que** le niveau de ladite arête (24) est distant de 5 à 50 mm de celui de l'extrémité aval du second canal d'amenée (12).

8. Chambre (1) de centrifugation selon la revendication 2, **caractérisée en ce que** l'arête (24) du déflecteur circulaire (20) est située à un niveau compris dans la moitié inférieure de la hauteur de la chambre de centrifugation.

9. Chambre (1) de centrifugation selon la revendication 8, **caractérisée en ce que** l'arête (24) du déflecteur circulaire (20) est située à un niveau compris dans le quart inférieur de la hauteur de la chambre de centrifugation.

10. Chambre (1) de centrifugation selon l'une des revendications 2 à 9, **caractérisée en ce que** la section annulaire de passage (25) est constituée d'une bague en matière poreuse.

## Claims

1. A method for washing and continuous separation of blood constituents using blood (11') and a sterile washing solution (12'), with a density lower than that of the blood (11'), which are introduced into a centrifugation chamber (1) provided with an inner cylindrical side wall (2) that rotates about an axis of revolution (5), **characterized in that** an axial tubular flow of blood (11') with a local thickness (e') is formed against the inner cylindrical side wall (2), and, between the latter and said axial tubular flow of blood (11'), a film of sterile washing solution (12') is formed with a thickness (e) substantially less than the local thickness (e'), flowing in the same axial direction as the tubular flow of blood (11') in such a way that, under the effect of the centrifugal force, this film gradually passes through the thickness (e') of blood (11') in the direction of the axis of revolution (5) of the chamber (1).

2. A centrifugation chamber (1) for washing and continuous separation of blood constituents, comprising an inner cylindrical side wall (2), a bottom (3) with an inner wall (4), an axis of revolution (5), and a circular opening (6) provided with a sealing means arranged around a static admission/evacuation member (10) extending into said chamber (1) at least partially along the axis of revolution (5) thereof and comprising at least one first intake channel (11) for the blood (11'), at least one second intake channel (12) for simultaneously conveying a sterile washing solution (12'), at least one first outlet channel (13) for at least one constituent (13') of this blood, and at least one second outlet channel (14) for a fluid (14') composed of said washing solution charged with waste materials and with the other blood constituents, **characterized in that** the second intake channel (12) has its downstream end continued on its periphery by a circular deflector (20), which extends in the bottom (3) of the chamber (1) at a distance from the inner wall (4) and is terminated along the inner cylindrical side wall (2) by a ridge (24) which defines, with this wall, a passage of annular cross section (25) for the flow of said sterile washing solution (12').

3. The centrifugation chamber (1) as claimed in claim 2, **characterized in that** the first intake channel (11) is annular and surrounds the second intake channel (12).

4. The centrifugation chamber (1) as claimed in claim 3, **characterized in that** the first and second intake channels (11, 12) are concentric.

5. The centrifugation chamber (1) as claimed in claim 2, **characterized in that** the passage of annular cross section (25) has a thickness (e) substantially less than the local thickness (e') of a layer of blood (11') rotating in the chamber (1) at the level of said ridge (24).

6. The centrifugation chamber (1) as claimed in claim 5, **characterized in that** the passage of annular cross section (25) has a thickness (e) of between 0.02 mm and 2 mm.

7. The centrifugation chamber (1) as claimed in claim 5, **characterized in that** the level of said ridge (24) is 5 to 50 mm away from that of the downstream end of the second intake channel (12).

8. The centrifugation chamber (1) as claimed in claim 2, **characterized in that** the ridge (24) of the circular deflector (20) is situated at a level located in the lower half of the height of the centrifugation chamber.

9. The centrifugation chamber (1) as claimed in claim 8, **characterized in that** the ridge (24) of the circular deflector (20) is situated at a level located in the lower quarter of the height of the centrifugation chamber.

10. The centrifugation chamber (1) as claimed in one of claims 2 through 9, **characterized in that** the passage of annular cross section (25) is formed by a ring made of porous material.

## Patentansprüche

1. Verfahren zum kontinuierlichen Waschen und Reinigen von Blutbestandteilen ausgehend von Blut (11') und einer sterilen Waschlösung (12') mit einer geringeren Dichte als der von Blut (11'), welche in eine Zentrifugierkammer (1) eingeführt sind, die mit einer zylinderförmigen inneren, sich um eine Drehachse (5) drehenden Seitenwand (2) ausgestattet ist, **dadurch gekennzeichnet, dass** ein röhrenartiger axialer und mit einer lokalen Dicke (e') ausgebildeter Abfluss von Blut (11') gegen die zylinderförmige innere Seitenwand (2) gebildet wird und zwischen dieser sowie dem röhrenartigen axialen Abfluss von Blut (11') eine Schicht einer sterilen Waschlösung (12') und einer Dicke (e) gebildet wird, die deutlich kleiner als die lokale Dicke (e') ist und die in die gleiche axiale Richtung wie der röhrenartige Auslass an Blut (11') ausströmt, so dass unter der Wirkung der Zentrifugalkraft diese Schicht nach und nach die Lage (e') an Blut (11') in Richtung der Drehachse (5) der Kammer (1) durchtritt.

2. Zentrifugierkammer (1) zum kontinuierlichen Waschen und Trennen von Blutbestandteilen mit einer zylinderförmigen inneren Seitenwand (2), mit einem Boden (3), der über eine Innenwand (4) verfügt, mit einer Drehachse (5) und mit einer kreisförmigen Öffnung (6), die mit einem Dichtmittel versehen ist, das um ein feststehendes Organ (10) zum Zuführen/Evakuieren angeordnet ist, welches sich wenigstens teilweise in die Kammer (1) entlang der Drehachse (5) erstreckt und wenigstens einen ersten Zuführkanal (11) für das Blut (11'), wenigstens einen zweiten Zuführkanal (12) zum gleichzeitigen Zuführen einer sterilen Reinigungslösung (12'), wenigstens einen ersten Auslasskanal (13) für wenigstens einen Bestandteil (13') des Bluts und wenigstens einen zweiten Auslasskanal (14) für ein Fluid (14') aufweist, das die mit Abfallstoffen und anderen Blutbestandteilen angereicherte Waschlösung bildet, **dadurch gekennzeichnet, dass** das in Strömungsrichtung hinten liegende Ende des zweiten Zuführkanals (12) an seinem Rand durch ein kreisförmiges Ablenkteil (20) verlängert ist, das sich zwischen dem Boden (3) der Kammer (1) in einem Abstand von der Innenwand (4) erstreckt und entlang der zylinderförmigen inneren Seitenwand (2) durch eine Kante (24) abgeschlossen ist, die mit dieser Wand einen kreisförmigen Durchlassabschnitt (25) zum Auslass der sterilen Waschlösung (12') bildet.

3. Zentrifugierkammer (1) nach Anspruch 2, **dadurch gekennzeichnet, dass** der erste Zuführkanal (11) kreisförmig ist und den zweiten Zuführkanal (12) umgibt.

4. Zentrifugierkammer (1) nach Anspruch 3, **dadurch gekennzeichnet, dass** die ersten und zweiten Zuführkanäle (11, 12) konzentrisch zueinander ausgerichtet sind.

5. Zentrifugierkammer (1) nach Anspruch 2, **dadurch gekennzeichnet, dass** der kreisförmige Durchlassabschnitt (25) eine Dicke (e) aufweist, die deutlich geringer als die lokale Dicke (e') einer Blutschicht (11') ist, die im Bereich der Kante (24) sich innerhalb der Kammer (1) in Drehung befindet.

6. Zentrifugierkammer (1) nach Anspruch 5, **dadurch gekennzeichnet, dass** der kreisförmige Durchlassabschnitt (25) eine Dicke (e) zwischen 0,02 mm und 2 mm aufweist.

7. Zentrifugierkammer (1) nach Anspruch 5, **dadurch gekennzeichnet, dass** der Bereich der Kante (24) von dem in Strömungsrichtung hinten liegenden Ende des zweiten Kanalauslasses (12) um 5 bis 50 mm beabstandet ist.

8. Zentrifugierkammer (1) nach Anspruch 2, **dadurch gekennzeichnet, dass** die Kante (24) des kreisförmigen Ablenkteiles (20) in einem Bereich angeordnet ist, der in der unteren Hälfte der Höhe der Zentrifugierkammer liegt.

9. Zentrifugierkammer (1) nach Anspruch 8, **dadurch gekennzeichnet, dass** die Kante (24) des kreisförmigen Ablenkteiles (20) in einem Bereich liegt, der in dem unteren Viertel der Höhe der Zentrifugierkammer liegt.

10. Zentrifugierkammer (1) nach einem der Ansprüche 2 bis 9, **dadurch gekennzeichnet, dass** der kreisförmige Durchlassabschnitt (25) durch einen Ring aus einem porösen Material gebildet ist.
